# FASCICULE DE BREVET EUROPEEN

(11) **EP 0 609 131 B1**
(45) Date de publication et mention de la délivrance du brevet: **17.12.1997**
(21) Numéro de dépôt: 94400148.6
(22) Date de dépôt: 25.01.1994
(51) Int. Cl.: A61K 7/48

(54) **Composition homogène à base de composés fluorés et de glycols, procédé de préparation et utilisation en cosmétique**
Homogene Zusammensetzung auf Basis von fluorierten Verbindungen und Glykolen, Verfahren zur Herstellung und Verwendung in der Kosmetik
Homogeneous composition based on fluorinated compounds and glycols, process of preparation and use in cosmetics

(30) Priorité: 25.01.1993 FR 9300912
(43) Date de publication de la demande: 03.08.1994
(73) Titulaire: L'OREAL, 75008 Paris (FR)
(72) Inventeur: Arnaud, Pascal, F-94000 Creteil (FR); Mellul, Myriam, F-94240 L'Hay Les Roses (FR)
(74) Mandataire: Casalonga, Axel

(56) Documents cités:
- EP-A- 0 051 527
- EP-A- 0 296 661
- EP-A- 0 390 206
- EP-A- 0 558 423
- WO-A-93/11103
- FR-A- 2 516 920

## Description

La présente invention concerne des compositions comportant un composé fluoré hydrocarboné ou un composé perfluoré en solution homogène avec un glycol, leur procédé de préparation et l'utilisation de ces solutions dans le domaine de la cosmétique et de la dermatologie.

En cosmétique, la prévention du vieillissement de la peau est un sujet d'étude constant et l'une des possibilités qui permet de ralentir le vieillissement cutané de la peau consiste à maintenir celle-ci dans un bon état d'hydratation. Pour atteindre ce but, de nombreuses compositions cosmétiques de soin et/ou de maquillage associent des composés hydratants avec un système lipophile sensé permettre la formation d'un film à la surface de la peau.

Ce film a pour but de limiter la perte d'eau transépidermique, ce qui suppose une certaine hydrophobicité mais aussi une certaine résistance vis-à-vis des sécrétions, comme la sueur ou le sébum, et vis-à-vis des agents extérieurs de l'environnement, et a par ailleurs pour but de permettre une bonne respiration de la peau, ce qui suppose une perméabilité vis-à-vis de l'oxygène et du gaz carbonique.

On a déjà proposé des huiles hydrocarbonées et des huiles de silicone, pour former ce film, car ces huiles sont hydrophobes; elles présentent toutefois l'inconvénient d'avoir une faible résistance vis-à-vis du sébum, ce qui conduit à une mauvaise tenue du film protecteur.

On a par ailleurs proposé des systèmes associant certaines substances hydrophiles avec des huiles fluorées; les huiles fluorées présentent en effet l'avantage de posséder un caractère très hydrophobe associé à une certaine lipophobicité. Il est par ailleurs connu, en particulier dans le domaine médical, que ces huiles sont capables de solubiliser des gaz comme l'oxygène et le gaz carbonique.

Ainsi, dans le brevet EP-196904, sont décrites des dispersions d'huiles perfluorées dans des émulsions; le caractère insoluble des huiles perfluorées, associé à leur densité plus forte que celle des autres constituants, mène toutefois à une distribution non homogène de ces huiles dans les préparations, ce qui entraîne une diminution des performances des produits cosmétiques.

Par ailleurs, certains documents décrivent l'émulsification de ces huiles fluorées sous la forme de systèmes eau-dans-huile ou huile-dans-eau, en présence d'un tensio-actif. Les brevets EP-390206 et EP-494412 décrivent des émulsions comportant des polyols du type glycérine et le brevet EP-296661 décrit des émulsions où un tensio-actif fluoré est utilisé.

Dans ces systèmes, la stabilité et les propriétés cosmétiques découlent de la présence du tensio-actif, ce qui est un facteur limitant dans le domaine de la cosmétique.

Il est donc apparu nécessaire de mettre au point une alternative à la présence des tensio-actifs pour obtenir une association compatible des huiles fluorées avec des substances hydrophiles sans qu'un système tensio-actif soit utile.

La demanderesse a maintenant mis au point des compositions homogènes en associant des glycols avec des composés fluorés. Ainsi, la présente invention concerne des solutions homogènes comportant au moins un composé organofluoré hydrocarboné ou un composé perfluoré associé à au moins un glycol hydrocarboné.

Selon l'invention, le terme organofluoré hydrocarboné désigne des composés dont la structure chimique comporte un squelette carboné où certains atomes d'hydrogène ont été substitués par des atomes de fluor, alors que le terme perfluoré désigne des composés où la substitution des atomes d'hydrogène liés au carbone par des atomes de fluor est totale. Le squelette hydrocarboné peut comporter un ou plusieurs hétéroatomes et/ou un ou plusieurs groupements organiques fonctionnels.

Pour les composés organofluorés hydrocarbonés, on définit le taux de substitution des atomes d'hydrogène par des atomes de fluor, sous la forme du rapport : nombre d'atomes de fluor/(nombre d'atomes de fluor + nombre d'atomes d'hydrogène) où seuls les atomes d'hydrogène liés aux atomes de carbone du squelette sont pris en compte. Les composés organofluorés hydrocarbonés ou les composés perfluorés de l'invention comportent au moins une fonction alcool, thiol, amine primaire ou secondaire. Les organofluorés sont non volatils, de point d'ébullition supérieur à 30°C.

Les composés organofluorés hydrocarbonés ou les composés perfluorés de l'invention ont pour formule la formule I suivante :

(R_{F})ₓ - (A)_{y} - (R_{H})_{z} (I)

dans laquelle :
x représente 1, 2 ou 3,
y représente 0 ou 1,
z représente 0, 1, 2 ou 3,
   à la condition que y et z ne soient pas simultanément 0, et que lorsque
z est 0, x est 2 ou 3,

le ou les radicaux fonctionnels OH, SH, NH₂ et H N peuvent se trouver indifféremment sur l'un ou plusieurs des radicaux RF, A, RH, dans une position intercalaire (NH), terminale ou pendante (OH, SH, NH₂). Lorsque y et z sont simultanément nuls, le radical RF comporte alors au moins un des radicaux fonctionnels précités,
R_{F} représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote et/ou substituée par des atomes d'hydrogène ou d'autres atomes d'halogène, à la condition que, pour deux atomes de carbone du squelette, ne soit pas présent plus d'un de ces substituants autres que le fluor,
R_{H} représente un radical hydrocarboné aliphatique ou aromatique, saturé ou insaturé, à chaîne linéaire, ramifiée ou cyclique, cette chaîne pouvant être fonctionnalisée et/ou interrompue par un ou plusieurs atomes divalents tels que l'oxygène ou le soufre ou par un ou plusieurs atomes trivalents comme l'azote, ce dernier étant alors totalement substitué.

A représente un radical di, tri ou quadrivalent tel que 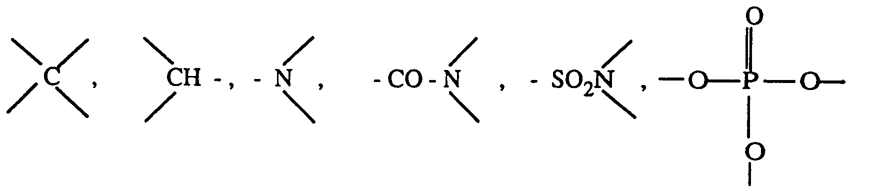 les structures cycliques, aliphatiques ou aromatiques ou les insaturations éthyléniques.

En plus des radicaux fonctionnels OH, SH, NH₂ et NH, RF et RH peuvent aussi contenir d'autres groupements organiques fonctionnels tels qu'une fonction acide, carbonyle, sulfoxyde, ester, amide, phosphate, énamine ou sulfonamide, en position intercalaire, terminale ou pendante.

Par insaturation éthylénique, on entend par exemple 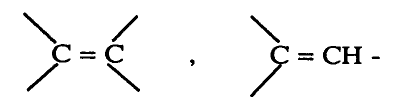 ou - CH = CH - .

De préférence, R_{H} représente un radical alkyle linéaire ou ramifié en C₁-C₂₂ ou un mélange de radicaux alkyle linéaires ou ramifiés en C₁-C₂₂, un radical aryle en C₆-C₁₀ ou un radical aralkyle en C₇-C₁₅.

De préférence, R_{F} représente un radical perfluoroalkyle ayant de 4 à 22 atomes de carbone.

Selon l'invention, le taux de substitution des atomes d'hydrogène liés au carbone par des atomes de fluor est compris entre 0,5 et 100%, et de préférence 10 à 100%.

A titre illustratif, on peut citer les composés organofluorés hydrocarbonés décrits dans la demande de brevet PCT/FR-92/01140 et dont la structure générale est définie par la formule (II) suivante :

R_{F} - (CH₂)ₙ- X - [C₃H₅(OH)] - (Y)ₓ - R_{H} (II)

dans laquelle C₃H₅(OH) représente les structures : 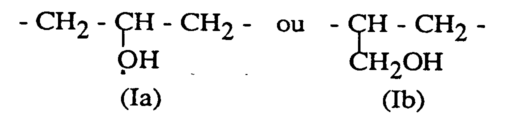
R_{H} représente un radical alkyle linéaire ou ramifié en C₁-C₂₂ ou un mélange de radicaux alkyle linéaires ou ramifiés en C₁-C₂₂ ou un radical aryle ou aralkyle;
R_{F} représente un radical perfluoroalkyle en C₄-C₂₂;
n est compris entre 0 et 4;
X représente O, S, 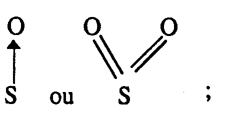
x représente O ou 1 ;
Y représente O, S, 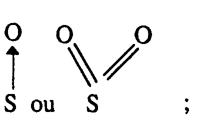 sous réserve que lorsque X = 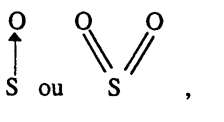
Y n'est pas

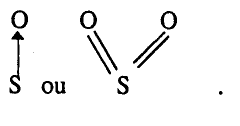

Ces composés peuvent être préparés en mettant en oeuvre la réaction d'un composé fluoré à hydrogène acide de formule :

R_{F} - (CH₂)ₙ - X - H

avec un époxyde de formule : 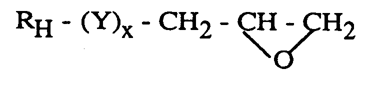 ou la réaction d'un composé hydrocarboné à hydrogène acide de formule :

R_{H} - (Y)ₓ - H

avec un époxyde fluoré de formule : 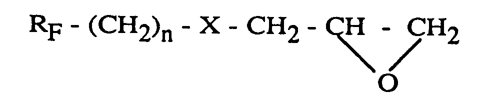 en présence d'un composé basique ou acide jouant le rôle de réactif ou de catalyseur, pour obtenir le composé de formule (II) correspondant, les substituants R_{F}, R_{H}, n et x ayant dans les formules ci-dessus la même signification que dans la formule (II) et X désignant O ou S, Y désignant O ou S, et en oxydant éventuellement la fonction mercaptan en sulfoxyde ou sulfone avec de l'eau oxygénée.

Ces composés sont décrits dans WO 93/11103 et EP-A-166.696.

Par ailleurs, on peut également utiliser, selon l'invention, les composés de formule (IV) :

R_{F} - (CH₂)ₙ - X - [C₃H₅ (OH)] - Y - (CH₂)ₘ - R'_{F} (IV)

dans laquelle C₃H₅ (OH) représente les structures : 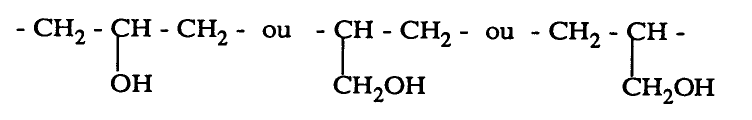
R_{F} et R'_{F}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4;
X et Y, identiques, sont - O - ou - S -.

Ces composés sont décrits dans DE-2.702.607, JP 89-193.236, JP 92-275.268 et US-3.893.984.

On peut utiliser aussi les composés de formule :

R_{F} - (CH₂)ₙ - X - [C₃H₅ (OH)] - Y - (CH₂)ₘ - R'_{F} (I')

dans laquelle C₃H₅ (OH) représente les structures : 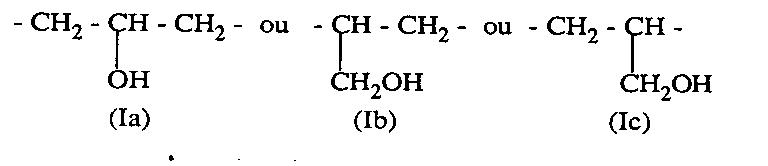
R_{F} et R'_{F}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4 et X est O et Y est S ou X est S et Y est O.

Les composés de formule (I') peuvent être préparés en mettant en oeuvre la réaction d'un composé fluoré à hydrogène acide de formule (II') :

R_{F} - (CH₂)ₙ - X - H (II')

avec un époxyde de formule (III') : 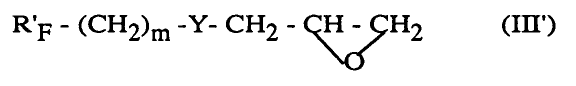 ou la réaction d'un composé fluoré à hydrogène acide de formule (IV') :

R_{F'} - (CH₂)ₘ - Y - H (IV')

avec un époxyde fluoré de formule (V') : 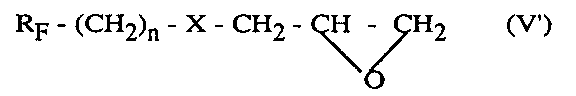 en présence d'un composé basique ou acide jouant le rôle de réactif ou de catalyseur. Les composés sont décrits dans FR 9306605.

On peut aussi utiliser, selon l'invention, les composés décrits dans le document US-3.952.066, de formule : 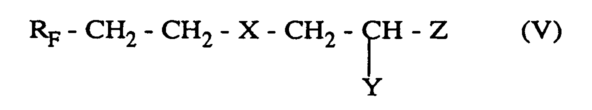
où Y est OH, et
Z est 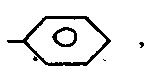 - CH₃, - CH₂OH, - CH₂OCOCH₃ ou bien Y est - CH₂OH et Z est -O-COCH₃
X représente - O - , - S -, 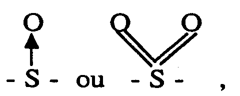 et
R_{F} représente un radical alkyle perfluoré, linéaire ou ramifié, en C₄-C₂₀, ou un mélange de radicaux alkyle perfluorés, linéaires ou ramifiés, en C₄-C₂₀;
   ou bien les composés décrits dans le document DE 2.052.579, de formule :

   R_{F} - CH = CH - CH₂ - O - CH₂ - [C₂H₄ - OW] (VI)

   où C₂H₄OW désigne : 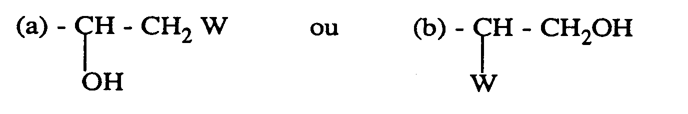
W désignant : -OR, -SR, -COOR, 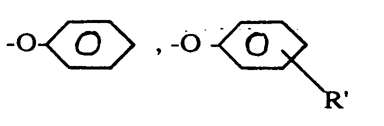
R désigne un radical alkyle en C₁-C₁₈, linéaire ou ramifié,
R' désigne -CH₃ ou -OH, en position ortho ou para, et
R_{F} représente un radical alkyle perfluoré, linéaire ou ramifié, en C₄-C₂₀, ou un mélange de radicaux alkyle perfluorés, linéaires ou ramifiés, en C₄-C₂₀.

Par ailleurs, on peut citer à titre d'exemple les produits vendus sous la dénomination de "FORALKYL EOH" par la Société ATOCHEM, ayant la formule suivante :

CₙF₂ₙ₊₁ - C₂H₄OH

dans laquelle n est 6 ou 8.

Les polyéthers fluorés fonctionnalisés ont la formule (III) :

R CF₂ - (OC₂F₄)ₚ - (OCF₂)_{q} - OCF₂ R (III)

dans laquelle p/q est de 0,5 à 1,5
et R représente CH₂OH,
CH₂(OCH₂CH₂)ₜOH où t est 1 ou 2, ou
CH₂OCH₂CHOHCH₂OH,
vendus respectivement sous les dénominations de "FOMBLIN ZDOL", "FOMBLIN ZDOL TX" et "FOMBLIN Z TETRAOL" par la Société MONTEFLUOS.

Selon l'invention, le ou les composés fluorés ainsi décrits représentent 0,1 à 99% en poids par rapport au poids total de la composition et de préférence 0,5 à 90% en poids.

Les solutions de l'invention contiennent un ou plusieurs polyols comportant deux radicaux hydroxyle encore appelés glycols. Les glycols utilisés selon l'invention ont un squelette hydrocarboné. Le squelette hydrocarboné peut être aliphatique ou aromatique, saturé ou insaturé, à chaîne linéaire ramifiée ou cyclique.

Les glycols hydrocarbonés peuvent avoir une masse moléculaire quelconque mais, de préférence, ils ont une longueur de chaîne comprise entre C₂ et C₃₀.

On fait le choix de la longueur de la chaîne en fonction du ou des composé(s) fluoré(s) associé(s) selon la miscibilité et les paramètres d'hydratation.

Les deux radicaux hydroxyle sont situés indépendamment l'un de l'autre en un endroit quelconque du squelette, en position pendante ou terminale.

A titre d'exemple, on peut citer les glycols hydrocarbonés tels que l'éthylèneglycol, le propylèneglycol, le diéthylèneglycol, le 1,3-butylèneglycol, le 3-méthyl 1,3-propanediol, l'isoprèneglycol, le néopentylglycol, le triéthylèneglycol, le dipropylèneglycol, l'héxylène glycol, le 2-éthyl 1,3-hexanediol, le 1,2-dihydroxybenzène, le résorcinol, le cyclohexanediméthanol, le styrèneglycol, le 2-butyl 2-éthyl 1,3-propanediol, le 1,2-dodécanediol, les polyéthylèneglycols, les polypropylèneglycols et les polybutylèneglycols.

On utilise de préférence les glycols hydrocarbonés en C₃-C₁₂.

Selon l'invention, le ou les glycol(s) représente(nt) 0,1 à 99% en poids par rapport au poids total de la composition, et de préférence 0,5 à 70% en poids.

Selon l'invention, lorsqu'on utilise des composés organofluorés hydrocarbonés de formule (II), les glycols comportent au moins 4 atomes de carbone.

Dans la solution constituée par le ou les composé(s) fluoré(s) et le ou les glycol(s), il est possible selon l'invention, d'introduire d'autres composés usuellement utilisés dans le domaine cosmétique à condition que ceux-ci soient solubilisés ou dispersés de manière homogène dans la solution initiale. Il peut s'agir de composés actifs mais aussi de composés usuellement utilisés à titre d'excipient.

Les additifs sont ajoutés en des quantités telles qu'il n'y ait pas démixtion entre le composé fluoré et le glycol.

A titre d'additif, on peut citer les huiles et les cires telles que :
- les huiles minérales telles que l'huile de paraffine, l'huile de vaseline et les huiles minérales ayant un point d'ébullition compris entre 310 et 410°C;
- les huiles d'origine animale telles que le perhydrosqualène;
- les huiles végétales telles que l'huile d'amande douce, l'huile de sésame, l'huile de calophyllum, l'huile de palme, l'huile d'avocat, l'huile de jojoba, l'huile d'olive, l'huile de ricin, les huiles de germes de céréales telles que l'huile de germes de blé;
- les esters de synthèse tels que l'huile de Purcelin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylène glycol, l'adipate de diisopropyle,
- les alcools gras tels que l'alcool oléique, l'alcool linoléique, l'alcool linolénique, l'alcool isostéarylique, l'octyl dodécanol,
- les esters dérivés d'acide lanolique tels que le lanolate d'isopropyle, le lanolate d'isocétyle,
- les acétylglycérides, les octanoates et décanoates d'alcools et de polyalcools tels que ceux de glycol et de glycérol, les ricinoléates d'alcools et de polyalcools tels que celui de cétyle,
- les cires minérales telles que les cires microcristallines, la paraffine, la vaseline, la cérésine,
- les cires fossiles telles que l'ozokérite, la cire de montan,
- les cires d'origine animale telles que la cire d'abeilles, le spermaceti, la cire de lanoline, les dérivés issus de la lanoline tels que les alcools de lanoline, la lanoline hydrogénée, la lanoline hydroxylée, la lanoline acétylée, les acides gras de la lanoline, l'alcool de lanoline acétylée,
- les cires d'origine végétale telles que la cire de candellila, la cire de Carnauba, la cire du Japon, le beurre de cacao,
- les cires synthétiques comme les cires de polyethylène,
- les huiles hydrogénées concrètes à 25°C telles que l'huile de ricin hydrogénée, l'huile de palme hydrogénée, le suif hydrogéné, l'huile de coco hydrogénée,
- les esters gras concrets à 25°C tels que le monomyristate de propylène glycol, le myristate de myristyle,
- parmi les cires, on peut également citer : l'alcool cétylique, l'alcool stéarylique, les mono-, di- et triglycérides concrets à 25°C, le monoéthanolamide stéarique, la colophane et ses dérivés tels que les abiétates de glycol et de glycérol, les sucro-glycérides et les oléates, myristates, lanolates, stéarates et dihydroxystéarates de calcium, magnésium, zinc et aluminium.

On peut également citer les composés siliconés suivants :
les diméthylpolysiloxanes cycliques, les diméthylpolysiloxanes de faible et/ou de haute viscosité, les gommes de silicone, des organopolysiloxanes comme les phénylméthylpolysiloxanes et les phényltriméthylsiloxypolysiloxanes, les alkylméthylpolysiloxanes, les alcoxyméthylpolysiloxanes, des silicones comportant des groupements fonctionnels comme les fonctions alcool ou amine ou thiol.

Par ailleurs on peut également citer les huiles perfluorées suivantes :
les huiles appartenant au groupe des perfluoroalcanes, des perfluorocycloalcanes, des perfluoropolycycloalcanes et des perfluoro (alkylcycloalcanes), mais aussi celles appartenant au groupe des hydrocarbures perfluorés aromatiques ou encore ceux appartenant au groupe des hydrocarbures perfluorés contenant au moins un hetéroatome comme les amines tertiaires, les composés hétérocycliques saturés ou enfin les perfluoropolyéthers.

On peut également incorporer des gélifiants de milieux huileux comme par exemple :
- les esters métalliques tels que le stéarate de polyoxyaluminium, ou l'hydroxystéarate d'aluminium et de magnésium,
- les esters d'acides gras et de glycol et les triglycérides,
- les mélanges d'alcools gras,
- les dérivés de cholestérol notamment l'hydroxycholestérol,
- des minéraux argileux gonflants avec les huiles appartenant au groupe des montmorillonites.

La solution peut en outre contenir des filtres, des vitamines, des hormones, des antioxydants, des conservateurs, des colorants, des parfums et tout additif lipophile habituellement utilisé en cosmétique.

Selon l'invention, la composition peut en outre contenir des additifs hydrophiles tels que :
. de l'eau , des polyols de fonctionnalité supérieure à 2, tels que : le glycérol et les polyglycérols, le 1,2,6-hexanetriol, le D-panthénol, le sorbitol, le mannitol, le xylitol, le maltitol, le glucose, le fructose, le sucrose et le saccharose;
. des actifs hydrophiles tels que le l'urée, l'acide lactique;
. des monoalcools tels que l'éthanol ou l'isopropanol;
. des gélifiantsde milieux aqueux comme :
   - les polysaccharides tels que les dérivés cellulosiques (carboxy méthylcellulose, hydroxypropylméthylcellulose, ...) et aussi la gomme de xanthane ou de caroube.
   - les protéines telles que la kératine, la kératine sulfonique, le collagène ou l'élastine.
   - les silicates tels que le silicate d'aluminium et de magnésium.
   - les dérivés acryliques tels que les Carbomer, les polyacrylates de glycérol et les polyméthacrylates de glycérol.
   - les polyéthylèneglycols.
. des actifs tels que le hyaluronate de sodium, le sel de sodium de l'acide pyroglutamique, le gluconate de magnésium, des oligo-éléments et des dérivés biologiques.
. des sels tels que le sulfate de magnésium ou le chlorure de sodium.
. des minéraux argileux gonflant en milieu aqueux comme la saponite, l'hectorite ou encore la smectite.
. des acides aminés, et
. des colorants.

Par ailleurs, les solutions de l'invention peuvent contenir des produits pulvérulents. A titre d'exemple, parmi les produits pulvérulents d'origine naturelle ou synthétique, on peut citer des poudres végétales telles que l'amidon de maïs, de froment ou de riz, des poudres minérales telles que le talc, le kaolin, le mica, la silice, les silicates, l'alumine, les zéolites, l'hydroxyapatite, la séricite, le dioxyde de titane, les micatitanes, l'oxyde de zinc, le sulfate de baryum, les oxydes de fer, le violet de manganèse, l'oxyde de chrome, le bleu d'outremer et l'oxychlorure de bismuth ou encore le nitrure de bore, des poudres métalliques telles que la poudre d'aluminium, des poudres organiques telles que les poudres de nylon, les poudres de polyamide, les poudres de polyester, les poudres de- cellulose, les poudres de polyéthylène, les poudres de polypropylène, les poudres de polystyrène, et les poudres de polytétrafluoroéthylène, et des pigments organométalliques associant le zirconium, le baryum ou l'aluminium à des colorants organiques.

Ces produits pulvérulents, utilisables dans les compositions de l'invention, peuvent être éventuellement enrobés par des sels métalliques d'acides gras, des acides aminés, de la lécithine, du collagène, du polyéthylène, des composés siliconés, des composés fluorés, des composés fluorosiliconés ou tout autre enrobage usuel.

La présente invention concerne également le procédé de préparation des compositions de l'invention. Le composé fluoré, d'une part, et le glycol, d'autre part, sont mélangés, dans le procédé selon l'invention, à une température dite "température de miscibilité" de façon à obtenir une seule phase homogène et stable à température ambiante.

Dans certains cas, le mélange peut être effectué à température ambiante. C'est le cas en particulier lorsque tous les constituants sont liquides ou que l'un ou l'autre d'entre eux peut permettre la solubilisation des autres.

Néanmoins, il est parfois préférable de chauffer, pour des raisons d'homogénéité, lorsque l'un ou plusieurs des constituants est dans un état solide ou cireux à température ambiante ou encore lorsque la dissolution est trop lente.

Les solutions ainsi obtenues peuvent se présenter à température ambiante sous différents aspects physiques : c'est-à-dire qu'elles peuvent être sous une forme liquide, pâteuse ou encore solide, l'aspect physique étant lié à la nature des constituants présents.

Les additifs, lorsqu'ils sont présents, sont solubilisés ou dispersés de manière homogène à différents stades de la préparation.

Ils peuvent être ainsi solubilisés ou dispersés à température ambiante ou à chaud dans la solution constituée par le ou les composés fluorés et le ou les glycols, ou dans chacun des constituants avant leur mélange pour former la solution.

Ces solutions possèdent de très bonnes propriétés sensorielles liées à la coexistence de composés fluorés avec des glycols au sein d'une même phase. Les compositions contenant ces solutions sont en effet très confortables, très faciles à appliquer et ont des textures très onctueuses.

Après application, elles laissent sur la peau un film particulièrement doux, protecteur, ayant une bonne tenue, et permettant une bonne hydratation.

Compte-tenu de ces propriétés et de la large gamme de compositions que l'on peut obtenir selon l'invention, les applications des solutions dans le domaine cosmétique et dermatologique sont multiples.

Ces solutions, selon l'invention, peuvent se présenter sous forme de lotions pour la peau ou les cheveux, de rouges à lèvres, de fonds de teint, de fards à paupières, de mascaras, de fard à joues coulé, de eye-liner.

Les solutions de l'invention ou les compositions cosmétiques préparées avec ces solutions, peuvent donc être utiles pour le traitement ou le soin de la peau, des cheveux et des cils.

D'autres caractéristiques et avantages de l'invention apparaîtront à la lecture des exemples ci-après.

### EXEMPLES 1 à 28

Des solutions sont préparées à partir des glycols hydrocarbonés suivants :
- Isoprèneglycol : (CH₃)₂CHOH-CH₂-CH₂OH;
- Hexylèneglycol : (CH₃)₂CHOH-CH₂-CHOH-CH₃.

Les composés fluorés suivants ont été testés pour leurs propriétés de solubiliser les glycols ci-dessus :
A : C₆F₁₃CH=CH₂ (FORALKYL E6 d'ATOCHEM)
B : 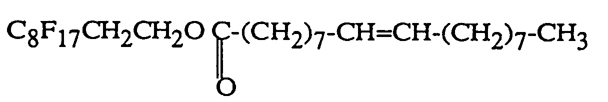 (NOFABLE FO 9982 de NIPPON OILS)
C : C₆F₁₃CH₂CH₂OH (FORALKYL EOH 6 d'ATOCHEM)
D : 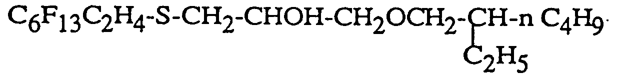
E : 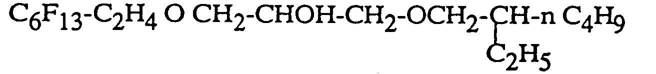
F : 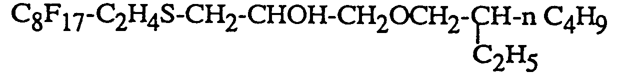
G : C₆F₁₃-C₂H₄-S-CH₂-CHOH-C₈H₁₇
H : C₈F₁₇-C₂H₄-S-CH₂-CHOH-CH₂ O - n C₄H₉
I : HOCH₂-(OC₂F₄)ₚ-(OCF₂)_{q}-CH₂OH (FOMBLIN Z DOL de MONTEFLUOS de poids moléculaire 2000)
J : HO(CH₂CH₂O)ₜ-CH₂-(OC₂F₄)ₚ-(OCF₂)_{q}-CH₂-(OCH₂CH₂)ₜOH (FOMBLIN Z DOL-TX de MONTEFLUOS de poids moléculaire 2200)
K : 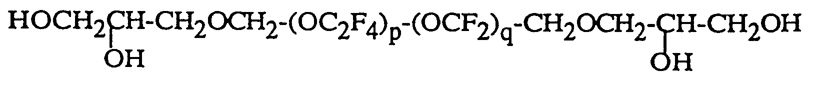 (FOMBLIN Z TETROL de MONTEFLUOS de poids moléculaire 1900)
L : C₈F₁₇-C₂H₄ NH₂
M : composé de l'exemple de préparation VI.
N : composé de l'exemple de préparation VII.

| Exemple | Composé fluoré | Isoprène glycol | Héxylène glycol |
|---|---|---|---|
| 1 et 2 | A | 0 | 0 |
| 3 et 4 | B | 0 | 3 % |
| 5 et 6 | C | ∞ | ∞ |
| 7 et 8 | D | 15 % | ∞ |
| 9 et 10 | E | 17 % | ∞ |
| 11 et 12 | F | 7,5 % | ∞ |
| 13 et 14 | G | 17 % | ∞ |
| 15 et 16 | H | 16 % | ∞ |
| 17 et 18 | I | 9 % | 8 % |
| 19 et 20 | J | 8 % | 18 % |
| 21 et 22 | K | 16 % | 16 % |
| 23 et 24 | L | ∞ | ∞ |
| 25 et 26 | M | 8 % | ∞ |
| 27 et 28 | N | 8 % | ∞ |

Le symbole ∞ signifie une miscibilité en toutes proportions.

Un pourcentage correspond au taux massique maximum de glycol que l'on peut solubiliser dans le composé fluoré considéré.

Les solubilités sont mesurées à 25°C en utilisant un microscope optique pour visualiser le nombre de phases.

Les exemples 1 à 4 sont comparatifs.

### EXEMPLE 29

On mélange 3,3 g d'hexylène glycol avec 3,4 g de composé fluoré D et 3,3 g d'octylméthoxycinnamate, vendu sous la dénomination de "PARSOL MCX" par la Société GIVAUDAN-ROURE.

Après homogénéisation, on obtient une solution limpide constituée d'une seule phase. Cette solution peut être utilisée comme lotion antisolaire.

### EXEMPLE 30

On mélange 6 g du composé D, 2 g d'hexylène glycol et 2 g de phényltriméthicone, vendue sous la dénomination de "DC556 Fluid" par la Société DOW CORNING.

Après homogénéisation, on obtient une solution limpide constituée d'une seule phase.

### EXEMPLE 31

On mélange 6 g du composé D, 2 g d'hexylène glycol et 2 g de perfluorodécaline, vendue sous la dénomination de "FLUTEC PP5" par la Société RHONE-POULENC.

Après homogénéisation, on obtient une solution limpide constituée d'une seule phase.

### EXEMPLE 32

On mélange 6 g d'hexylène glycol avec 2 g du composé fluoré D et 2 g de glycérol.

Après homogénéisation, on obtient une solution limpide constituée d'une seule phase. Cette lotion peut être utilisée comme lotion hydratante.

### EXEMPLE 33

On mélange 8 g d'hexylène glycol avec 1 g d'eau et 1 g du composé fluoré D.

Après homogénéisation, on obtient une solution limpide constituée d'une seule phase. Cette lotion peut être utilisée comme lotion hydratante.

### EXEMPLE 34

On prépare une solution de 5 g d'urée dans 5 g d'eau.

Après dissolution, on prélève 1 g de cette solution que l'on mélange avec 8 g d'hexylène glycol et 1 g de composé fluoré D.

Après homogénéisation, on obtient une solution limpide constituée d'une seule phase qui peut être utilisée comme lotion hydratante.

### EXEMPLE 35

On mélange 2 g d'hexylèneglycol avec 8 g de F-hexyléthanethiol.

Après homogénéisation, on obtient une solution limpide constituée d'une seule phase.

### EXEMPLE 36 : Lotion démaquillante

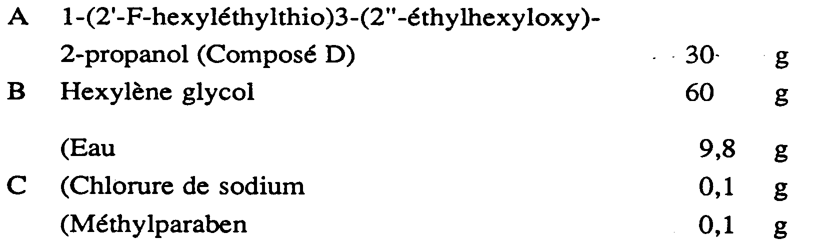

### Mode opératoire

Les constituants de la phase C sont pesés ensemble puis chauffés à 80°C jusqu'à dissolution complète.

Après refroidissement à l'ambiante, on ajoute l'hexylène glycol et le composé fluoré.

Après homogénéisation, on obtient une lotion constituée d'une seule phase limpide qui peut être utilisée comme démaquillant et qui laisse après application un toucher très doux dû à la présence de l'huile fluorée.

### EXEMPLE 37 : Rouge à lèvres

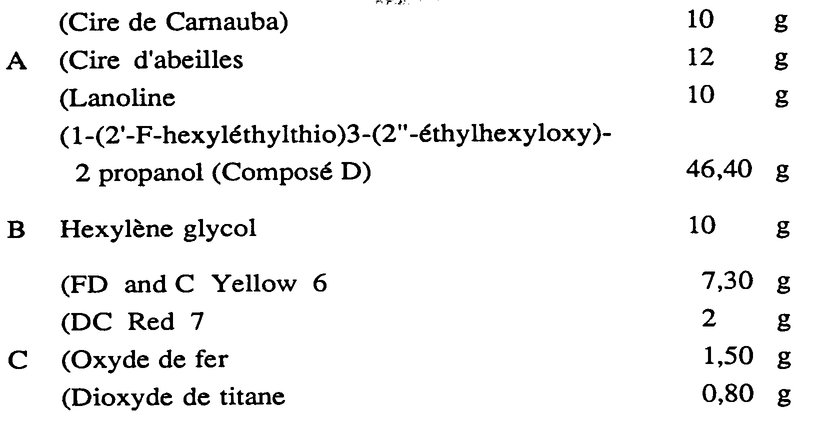

### Mode opératoire

On pèse ensemble les constituants de la phase A qui est ensuite chauffée à 90°C jusqu'à obtention d'un milieu limpide. On ajoute ensuite la phase C.

Après avoir diminué la température à 80°C, on ajoute la phase B.

Après homogénéisation, on coule la pâte à 80°C dans des moules.

Le rouge à lèvres ainsi obtenu est facile à appliquer, très doux et possède des propriétés hydratantes.

### EXEMPLE 38 : Fard à paupières

| | |
|---|---|
| - 1-(2'-F-hexyléthylthio)3-(2''-éthylhexyloxy)-2 propanol | 5,85 g |
| - Isoprèneglycol | 0,65 g |
| - Oxydes de fer | 9,10 g |
| - Oxyde de chrome | 5,40 g |
| - Dioxyde de titane | 2 g |
| - Mica | 22 g |
| - Poudre de nylon | 20 g |
| - Talc | |
| | qsp 100 g |

### EXEMPLES DE PREPARATION

### EXEMPLE I

### 1-(2'-F-hexyléthylthio) 3-(2''-éthylhexyloxy)-2-propanol

A la température de 25°C, sous agitation et sous courant d'azote, on ajoute à 152 g de 2-F-hexyléthanethiol, 3,6 g d'une solution méthanolique de méthylate de sodium (environ 30% - 5,54 meq g⁻¹) en une minute.

Le mélange est chauffé à 70°C. On évapore sous vide le méthanol présent dans le milieu.

Le 2-éthylhexylglycidyléther (74,4 g) est ensuite ajouté goutte-à-goutte en une heure. On maintient la température du mélange entre 60 et 70°C au cours de l'addition de l'époxyde.

A la fin de l'addition, la température est amenée à 25°C.

Le mélange est neutralisé à l'aide de 20 ml de HCl Normal.

Le 1-(2'-F-hexyléthylthio)3-(2''-éthylhexyloxy)2-propanol est séparé par distillation : Eb = 141°C/66,5 Pa.

On obtient 175 g (77%) d'une huile translucide incolore.

| Analyse élémentaire : | | | | |
|---|---|---|---|---|
| | % C | % H | % S | % F |
| Calculé | 40,28 | 4,80 | 5,66 | 43,60 |
| Mesuré | 40,37 | 4,82 | 5,55 | 43,74 |

### EXEMPLE II

### 1-(2'-F-octyléthylthio)3-(2''-éthylhexyloxy)-2-propanol

Le composé est préparé de façon analogue à la préparation décrite à l'exemple I où l'on utilise :
- 288 g de 2-F-octyléthanethiol
- 5,4 g d'une solution méthanolique de méthylate de sodium (5,54 meq g⁻¹)
- 111,6 g de 2-éthylhexylglycidyléther
- 30 ml de HCl normal

On obtient 337 g (81%) d'une huile translucide incolore.

| Analyse élémentaire : | | | | |
|---|---|---|---|---|
| | % C | % H | % S | % F |
| Calculé | 37,84 | 4,08 | 4,81 | 48,46 |
| Mesuré | 37,83 | 4,06 | 4,20 | 47,45 |

### EXEMPLE III

### 1-(2'-F-octyléthylthio)3-butyloxy 2-propanol

Selon le mode opératoire décrit à l'exemple I, on condense en 1 heure 40 g (0,31 mole) d'éther de butyle et de glycidyle sur 147,7 g (0,31 mole) de 2-F-octyléthanethiol, en présence de 2,75 g de solution méthanolique de méthylate de sodium (5,54 meq g⁻¹). En fin de réaction, le mélange est neutralisé par 15,5 ml de HCl Normal.

Après distillation (138° - 142°C/6,65 Pa), on obtient 153 g de 1-(2'-F-octyléthylthio)3-butyloxy 2-propanol sous forme d'une huile incolore.

Rendement = 80%.

| Analyse élémentaire : | | | | |
|---|---|---|---|---|
| | % C | % H | % S | % F |
| Calculé | 33,45 | 3,14 | 5,25 | 52,92 |
| Mesuré | 33,52 | 3,23 | 5,14 | 52,67 |

### EXEMPLE IV

### 1-(2'-F-hexyléthylthio)2-décanol

Selon le mode opératoire décrit à l'exemple I, on condense en 1 heure 30 minutes 78 g (0,5 mole) de 1,2-époxydécane avec 130 g (0,5 mole) de 2-F-hexyléthanethiol en présence de 4,4 g de solution méthanolique de méthylate de sodium (5,65 meq g⁻¹).

En fin de réaction, le mélange est neutralisé par 25 ml de HCl Normal.

Après distillation (165-170°C/66,5 Pa), on obtient 216 g d'un solide amorphe de couleur blanche qui est le 1-(2'-F-hexyléthylthio) 2-décanol.

Rendement = 81%.

Point de fusion = 47°C.

| Analyse élémentaire : | | | | |
|---|---|---|---|---|
| | % C | % H | % S | % F |
| Calculé | 40,30 | 4,70 | 5,98 | 46,04 |
| Mesuré | 40,06 | 4,62 | 6,13 | 45,63 |

### EXEMPLE V

### 1-(2'-F-hexyléthyloxy)-3-(2''-éthylhexyloxy)-2-propanol

Dans un réacteur de 1 litre, on introduit 546 g (1,5 mole) de 2-F-hexyléthanol.

On ajoute à 25°C et sous atmosphère d'azote 5,61 g de tertiobutylate de potassium. Le mélange est agité à 25°C pendant 30 minutes pour solubiliser le tertiobutylate dans le 2-F-hexyléthanol.

Le mélange est porté à 150°C et on additionne en 75 minutes 93 g (0,5 mole) de 2-éthylhexylglycidyléther.

Après 24 heures de réaction à 150°C, on ajoute 5,61 g de tertiobutylate de potassium. On renouvelle cette opération deux fois à des intervalles de temps de 24 heures.

Le 2-F-hexyléthanol en excès est alors évaporé et on obtient par distillation 68 g (20%) de 1-(2'-F-hexyléthoxy)-3-(2''-éthylhexyloxy)-2-propanol.

Point d'ébullition = 145°C sous 13,3 Pa.

| Analyse élémentaire : | | | |
|---|---|---|---|
| | % C | % H | % F |
| Calculé | 41,46 | 4,94 | 44,87 |
| Mesuré | 41,55 | 4,99 | 45,01 |

### EXEMPLE VI

### 1-(2'-F-hexyléthylthio)-3-(2'-F-hexyléthoxy)-2-propanol

A la température de 25°C, sous agitation et sous courant d'azote, on ajoute 1,33 g d'une solution méthanolique de méthylate de sodium (environ 30% - 5,65 meq g⁻¹) à 57 g de 2-F-hexyléthanethiol, en une minute.

Le mélange est chauffé à 70°C. On évapore sous vide le méthanol présent dans le milieu.

Le 2-F-hexyléthylglycidyléther (63 g - 0,15 mole) est ensuite ajouté goutte-à-goutte en une heure. On maintient la température du mélange entre 60 et 70°C au cours de l'addition de l'époxyde.

A la fin de l'addition, la température est amenée à 25°C.

Le mélange est neutralisé à l'aide de 7,5 ml de HCl Normal.

Le 1-(2'-F-hexyléthylthio)-3-(2'-F-hexyléthoxy)-2-propanol est séparé par distillation : Eb = 170°C/133 Pa.

On obtient 85 g (71%) d'une huile translucide incolore.

| Analyse élémentaire : | | | | |
|---|---|---|---|---|
| | % C | % H | % S | % F |
| Calculé | 28,51 | 1,76 | 4,01 | 61,72 |
| Mesuré | 28,60 | 1,79 | 4,32 | 61,54 |

### EXEMPLE VII

### 1-(2'-F-hexyléthylthio)-3-octylthio-2-propanol

A la température de 25°C et sous courant d'azote, on ajoute 0,61 g d'une solution méthanolique de méthylate de sodium (environ 30% - 5,65 meq g⁻¹) à 10,05 g (0,069 mole) d'octanethiol.

Le mélange est chauffé à 70°C. On évapore sous vide le méthanol présent dans le milieu.

Le 2-F-hexyléthylthioglycidyléther (30 g - 0,069 mole) est ensuite ajouté goutte-à-goutte en 30 minutes. On maintient la température du mélange entre 60 et 70°C au cours de l'addition de l'époxyde.

A la fin de l'addition, la température est amenée à 25°C.

Le mélange est neutralisé à l'aide de 3,5 ml de HCl Normal.

Le 1-(2'-F-hexyléthylthio)-3-octylthio-2-propanol est séparé par distillation : Eb = 178°C/66,5 Pa.

On obtient 30 g (75%) d'une huile transparente incolore.

| Analyse élémentaire : | | | | |
|---|---|---|---|---|
| | % C | % H | % S | % F |
| Calculé | 39,18 | 4,67 | 11,01 | 42,40 |
| Mesuré | 39,16 | 4,65 | 10,57 | 42,46 |

## Revendications

1. Composition homogène comportant :
- de 0,1 à 99% en poids par rapport au poids total de la composition, et de préférence 0,5 à 90% en poids, d'au moins un composé organofluoré hydrocarboné ou d'un composé perfluoré carboné possédant au moins une fonction alcool, thiol, amine primaire ou secondaire, et
- de 0,1 à 99% en poids par rapport au poids total de la composition, et de préférence de 0,5 à 70% en poids, d'au moins un glycol hydrocarboné comportant deux radicaux hydroxyle.

2. Composition selon la revendication 1, caractérisée en ce que le taux de substitution des composés fluorés est compris entre 0,5 et 100%, et de préférence compris entre 10 et 100%.

3. Composition selon l'une des revendications 1 ou 2, caractérisée en ce que le composé fluoré a pour formule (I) :
(R_{F})ₓ- (A)_{y} - (R_{H})_{z} (I)
dans laquelle :
x représente 1, 2 ou 3,
y représente 0 ou 1,
z représente 0, 1, 2 ou 3,
à la condition que y et z ne soient pas simultanément 0, et que lorsque z est 0, x est 2 ou 3,
R_{F} représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote et/ou substituée par des atomes d'hydrogène ou d'autres atomes d'halogène, à la condition que, pour deux atomes de carbone du squelette, ne soit pas présent plus d'un de ces substituants autres que le fluor,
R_{H} représente un radical hydrocarboné aliphatique ou aromatique, saturé ou insaturé, à chaîne linéaire, ramifiée ou cyclique, cette chaîne pouvant être. fonctionnalisée et/ou interrompue par un ou plusieurs atomes divalents tels que l'oxygène ou le soufre ou par un ou plusieurs atomes trivalents comme l'azote,
A représente un radical di, tri ou quadrivalent tel que 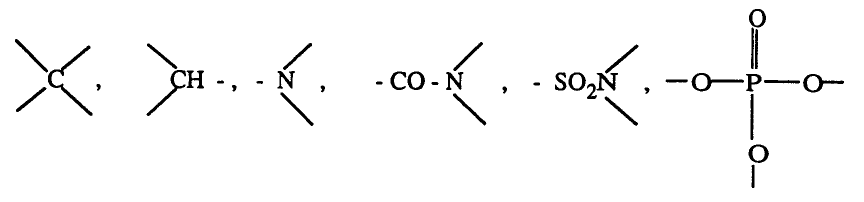 les structures cycliques, aliphatiques ou aromatiques ou les insaturations éthyléniques

4. Composition selon l'une des revendications 1 à 3, caractérisée en ce que les composés organofluorés hydrocarbonés ont la formule (II) suivante :
R_{F} - (CH₂)ₙ - X - [C₃H₅(OH)] - (Y)ₓ - R_{H} (II)
dans laquelle C₃H₅(OH) représente les structures : 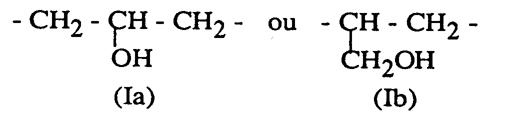
R_{H} représente un radical alkyle linéaire ou ramifié en C₁-C₂₂ ou un mélange de radicaux alkyle linéaires ou ramifiés en C₁-C₂₂ ou un radical aryle ou aralkyle;
R_{F} représente un radical perfluoroalkyle en C₄-C₂₂;
n est compris entre 0 et 4;
X représente O, S, 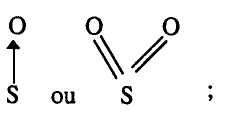
x représente 0 ou 1 ; 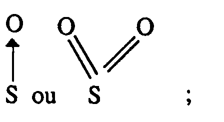
Y représente O, S, 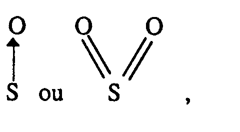 sous réserve que lorsque X =
Y n'est pas 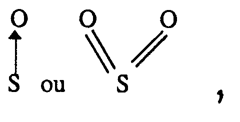 ou de formule (I') :
R_{F} - (CH₂)ₙ - X - [C₃H₅ (OH)] - Y - (CH₂)ₘ - R'_{F} (I')
dans laquelle C₃H₅ (OH) représente les structures : 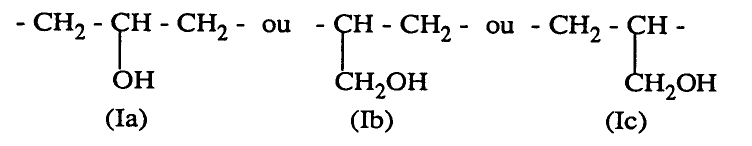
R_{F} et R'_{F}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4 et X est O et Y est S ou X est S et Y est O.

5. Composition selon l'une des revendications 1 à 4, caractérisée en ce que le glycol hydrocarboné a une chaîne comportant 2 à 30 atomes de carbone.

6. Composition selon l'une des revendications 1 à 5, caractérisée en ce que le glycol hydrocarboné comporte de 3 à 12 atomes de carbone.

7. Composition selon l'une des revendications 1 à 6, caractérisée en ce qu'eue comporte au moins un composé organofluoré hydrocarboné selon la revendication 4 et au moins un glycol comportant au moins 4 atomes de carbone.

8. Composition selon l'une des revendications 1 à 7, caractérisée par le fait qu'elle comporte au moins un additif choisi parmi les huiles, les cires, les silicones, les huiles perfluorées, les gélifiants de milieux huileux, les gélifiants de milieu aqueux, l'eau, les polyols ayant au moins trois groupements hydroxyle, les monoalcools, l'urée, l'acide lactique, les sels, les filtres, les vitamines, les hormones, les antioxydants, les conservateurs, les colorants, les parfums, les acides aminés, les produits pulvérulents.

9. Procédé de préparation d'une composition selon l'une des revendications 1 à 8, caractérisé en ce que l'on mélange des constituants à la température de miscibilité, pour obtenir une phase homogène et des excipients éventuels sont solubilisés ou dispersés dans cette phase homogène.

10. Utilisation des compositions selon l'une des revendications 1 à 8, pour la préparation de compositions cosmétiques ou dermatologiques.

## Claims

1. Homogeneous composition comprising:
- from 0.1 to 99%, preferably 0.5 to 90% by weight with respect to the total composition weight of at least one organofluorinated hydrocarbon compound or a perfluorinated carbon compound having at least one alcohol, thiol, primary or secondary amine functional group, and
- from 0.1 to 99%, preferably 0.5 to 70% by weight with respect to the total composition weight of at least one hydrocarbon glycol including two hydroxyl radicals.

2. Composition according to claim 1 characterized in that the substitution ratio for the fluorinated compounds is between 0.5 and 100%, preferably between 10 and 100%.

3. Composition according to claim 1 or claim 2 characterized in that the fluorinated compound has the following formula (I):
(R_{F})ₓ - (A)_{y} - (R_{H})_{z} (I)
wherein:
x is 1, 2 or 3,
y is 0 or 1,
z is 0, 1, 2 or 3,
provided that y and z are not simultaneously 0 and that
when z is 0 x is 2 or 3,
R_{F} is an aliphatic or aromatic, saturated or unsaturated fluorinated radical with a linear, branched or cyclic chain capable of being functionalized and/or interrupted by divalent atoms such as oxygen or sulfur or trivalent atoms such as nitrogen and/or substituted by hydrogen atoms or other halogen atoms provided that, for two carbon atoms of the backbone, no more than one of these substituents other than fluorine is present,
R_{H} is an aliphatic or aromatic, saturated or unsaturated hydrocarbonated radical, with a linear, branched or cyclic chain capable of being functionalized and/or interrupted by one or more divalent atoms such as oxygen or sulfur or by one or more trivalent atoms such as nitrogen,
A is a di-, tri- or quadrivalent radical such as 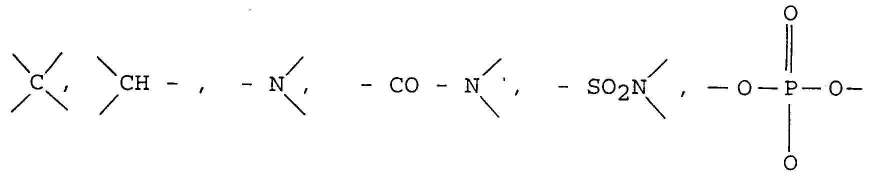 cyclic, aliphatic or aromatic structures or unsaturated ethylene structures.

4. Composition according to any one of claims 1 to 3 characterized in that the organofluorinated hydrocarbon compounds have the following formula (II):
R_{F} - (CH₂)ₙ - X - [C₃H₅(OH)] - (Y)ₓ - R_{H} (II)
wherein C₃H₅(OH) is the structure: 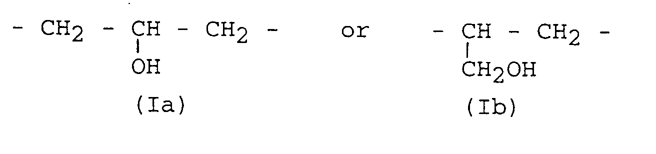
R_{H} is a linear or branched C₁-C₂₂ alkyl radical or a mixture of linear or branched C₁-C₂₂ alkyl radicals or an aryl or aralkyl radical;
R_{F} is a C₄-C₂₂ perfluoroalkyl radical;
n is between 0 and 4;
X is O, S, 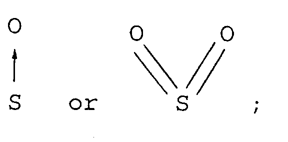
x is 0 or 1;
Y is O, S, 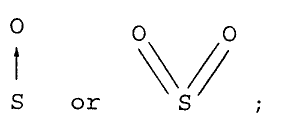 provided that when X = 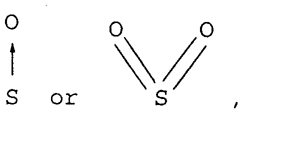
Y is not 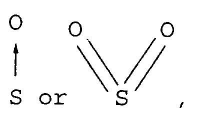 or formula (I'):
R_{F} - (CH₂)ₙ - X - [C₃H₅(OH)] - Y - (CH₂)ₘ - R'_{F} (I')
in which C₃H₅(OH) represents the structures: 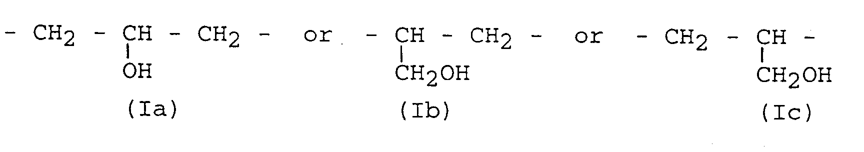
R_{F} and R'_{F}, which are identical or different, represent a linear or branched perfluorinated C₄-C₂₀ alkyl radical or a mixture of linear or branched perfluorinated C₄-C₂₀ alkyl radicals;
m and n, which are identical or different, represent 0, 1, 2, 3 or 4 and X is O and Y is S or X is S and Y is O.

5. Composition according to any one of claims 1 to 4 characterized in that the hydrocarbon glycol comprises a chain having 2 to 30 carbon atoms.

6. Composition according to any one of claims 1 to 5 characterized in that the hydrocarbon glycol comprises 3 to 12 carbon atoms.

7. Composition according to any one of claims 1 to 6 characterized in that it comprises at least one glycol comprising at least four carbon atoms and at least one organofluorinated hydrocarbon compound as claimed in claim 4.

8. Composition according to any one of claims 1 to 7 characterized in that it comprises at least one additive selected from oils, waxes, silicones, perfluorinated oils, gelling agents for oily media, gelling agents for aqueous media, water, polyols having at least three hydroxyl groups, monoalcohols, urea, lactic acid, salts, filters, vitamins, hormones, antioxidants, preservatives, dyes, perfumes, amino acids or powders.

9. Method of preparing a composition as claimed in any one of claims 1 to 8 characterized in that the constituents are mixed at the miscibility temperature to produce a homogeneous phase and any excipients are dissolved or dispersed in this homogeneous phase.

10. Use in the preparation of cosmetic or dermatological compositions of compositions as claimed in any one of claims 1 to 8.

## Patentansprüche

1. Homogene Zusammensetzung, enthaltend:
- 0,1 bis 99 und vorzugsweise 0,5 bis 90 Gew.%, bezogen auf das Gesamtgewicht der Zusammensetzung, mindestens einer Organofluorkohlenwasserstoffverbindung oder einer prfluorierten Kohlenstoffverbindung, welche mindestens eine Alkohol-, Thiol- und primäre oder sekundäre Amin-Funktion enthalten, sowie
- 0,1 bis 99 und vorzugsweise 0,5 bis 70 Gew.%, bezogen auf das Gesamtgewicht der Zusammensetzung, mindestens einer Kohlenwasserstoffglycolverbindung mit 2 Hydroxylresten.

2. Zusammensetzung gemäß Anspruch 1,
dadurch **gekennzeichnet**, daß
der Substitutionsgehalt der Fluorverbindungen 0,5 bis 100 und vorzugsweise 10 bis 100% beträgt.

3. Zusammensetzung gemäß Anspruch 1 oder 2,
dadurch **gekennzeichnet**, daß
die Fluorverbindung die Formel (I) aufweist:
(R_{F})ₓ - (A)_{y} - (R_{H})_{z} (I)
worin gilt:
X stellt 1, 2 oder 3 dar,
y stellt 0 oder 1 dar,
z stellt 0, 1, 2 oder 3 dar,
mit der Maßgabe, daß y und z nicht gleichzeitig 0 sind, und daß, wenn z 0 ist, x 2 oder 3 ist,
R_{F} stellt einen aliphatischen oder aromatischen, gesättigten oder ungestättigten fluorhaltigen Rest mit linearer, verzweigter oder zyklischer Kette dar, wobei diese Kette funktionalisiert und/oder mit zweiwertigen Atomen wie Sauerstoff oder Schwefel oder mit dreiwertigen Atomen wie Stickstoff unterbrochen und/oder mit Wasserstoffatomen oder anderen Halogenatomen substituiert sein kann, mit der Maßgabe, daß, für zwei Kohlenstoffatome des Skeletts, nicht mehr als einer dieser von Fluor verschiedenen Substituenten vorliegt,
R_{H} stellt einen aliphatischen oder aromatischen, gesättigten oder ungesättigten Kohlenwasserstoffrest mit linearer, verzweigter oder zyklischer Kette dar, wobei diese Kette funktionalisiert und/oder mit einem oder mehreren zweiwertigen Atomen wie Sauerstoff oder Schwefel oder mit einem oder mehreren dreiwertigen Atomen wie Stickstoff unterbrochen sein kann,
A stellt einen zwei-, drei- oder vierwertigen Rest wie: 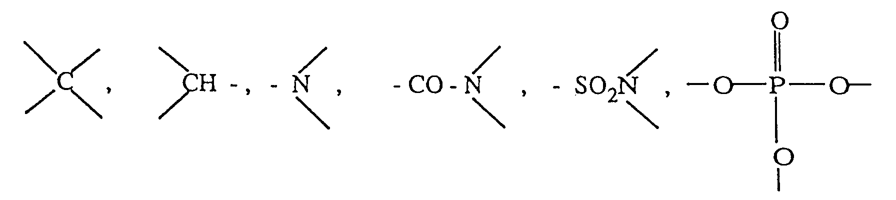 zyklische, aliphaische oder aromatische oder ethylenisch ngesättigte Strukturen dar.

4. Zusammensetzung gemäß einem der Ansprüche 1 bis 3,
dadurch **gekennzeichnet**, daß
die Organofluorkohlenwasserstoffverbindungen die folgende Formel (II) aufweisen:
R_{F} - (CH₂)ₙ - X - [C₃H₅(OH)] - (Y)ₓ - R_{H} (II)
worin gilt: C₃H₅(OH) stellt die Strukturen dar: 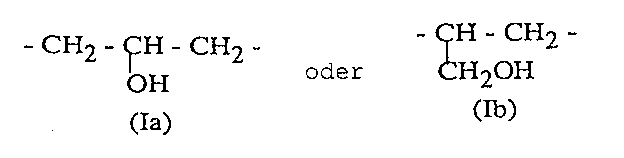
R_{H} stellt einen linearen oder verzweigten C₁₋₂₂-Alkylrest oder eine Mischung aus linearen oder verzweigten C₁₋₂₂-Alkylresten oder einen Aryl- oder Aralkylrest dar;
R_{F} stellt einen C₄₋₂₂-Perfluoralkylrest dar;
n beträgt 0 bis 4;
X stellt O, S, 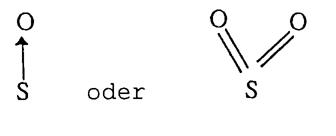 dar;
x stellt 0 oder 1 dar;
Y stellt O, S, 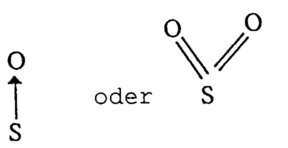 dar;
mit der Maßgabe, daß, wenn X = 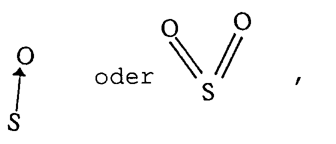
Y nicht 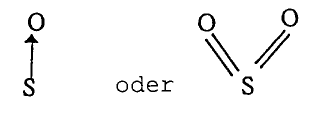 ist, oder daß die Verbindungen die Formel (I') aufweisen:
worin gilt:
R_{F} - (CH₂)ₙ - X - [C₃H₅(OH)] - Y - (CH₂)ₘ - R'_{F} (I')
worin gilt: C₃H₅(OH) stellt die Strukturen dar: 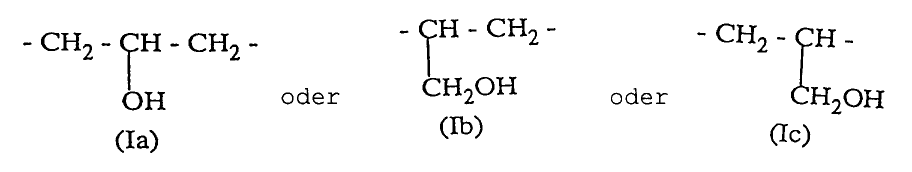
R_{F} und R'_{F} stellen, gleich oder verschieden, einen linearen oder verzweigten C₄₋₂₀-Perfluoralkylrest oder eine Mischung aus linearen oder verzweigten C₄₋₂₀-Perfluoralkylresten dar;
m und n stellen, gleich oder verschieden, 0, 1, 2, 3 oder 4 dar; und X ist O und Y ist S, oder X ist S und Y ist O.

5. Zusammensetzung gemäß einem der Ansprüche 1 bis 4,
dadurch **gekennzeichnet**, daß
da Kolenwasserstoffglycol eine Kette mit 2 bis 30 Kohlenstoffatomen aufweist.

6. Zusammensetzung gemäß einem der Ansprüche 1 bis 5,
dadurch **gekennzeichnet**, daß
das Kohlenwasserstoffglycol 3 bis 12 Kohlenstoffatome aufweist.

7. Zusammensetzung gemäß einem der Ansprüche 1 bis 6,
dadurch **gekennzeichnet**, daß
sie mindestens eine in Anspruch 4 definierte Organofluorkohlenwasserstoffverbindung und mindestens ein Glycol enthält, das mindestens 4 Kohlenstoffatome aufweist.

8. Zusammensetzung gemäß einem der Ansprüche 1 bis 7,
dadurch **gekennzeichnet**, daß
sie mindestens ein Additiv enthält, ausgewählt aus Ölen, Wachsen, Siliconen, perfluorierten Ölen, Geliermitteln für ölartige Medien, Geliermitteln für wässriges Medium, Wasser, Polyolen mit mindestens drei Hydroxylgruppen, Monoalkoholen, Harnstoff, Milchsäure, Salzen, Filterstoffverbindungen, Vitaminen, Hormonen, Antioxidantien, Konservierungsmitteln, Färbemitteln, Parfüm-Produkten, aminierten Säuren und aus pulvrigen Produkten.

9. Verfahren zur Herstellung einer Zusammensetzung gemäß einem der Ansprüche 1 bis 8,
dadurch **gekennzeichnet**, daß
man die Bestandteile bei der Vermischbarkeitstemperatur vermischt, um eine homogene Phase zu erhalten, und daß gegebenenfalls vorhandene Exzipienten in dieser homogenen Phase solubilisiert oder dispergiert werden oder sind.

10. Verwendung der Zusammensetzungen gemäß einem der Ansprüche 1 bis 8 zur Herstellung von kosmetischen oder dermatologischen Zusammensetzungen.
